# EUROPÄISCHE PATENTANMELDUNG

(11) **EP 3 989 137 A1**
(43) Veröffentlichungstag der Anmeldung: **27.04.2022**
(21) Anmeldenummer: 20202940.1
(22) Anmeldetag: 21.10.2020
(51) Int. Cl.: G06Q 10/06, A61B 90/00, G06F 21/64, H04L 29/08

(54) **SICHERSTELLUNG SINGLE USE ANWENDUNGEN VON SU PRODUKTEN MITTELS DLT/BLOCKCHAIN**

(71) Anmelder: Aesculap AG, 78532 Tuttlingen (DE)
(72) Erfinder: Lenzenhuber, Frederick, 78351 Tuttlingen (DE)

(57) **Zusammenfassung**

Die vorliegende Erfindung betrifft ein computer-implementiertes Verfahren, eine Vorrichtung und ein System zur Speicherung von Nutzungsdaten eines Produktes, insbesondere eines Medizinprodukts, mit Erfassen einer Nutzung des Produktes, Erstellen der Nutzungsdaten, die die erfasste Nutzung des Produktes betreffen, und Speichern der Nutzungsdaten in einem Datensatz in einer nachträglich nicht modifizierbaren Form, und zwar in einer Blockchain. Ein entsprechendes System zur Datenverarbeitung, ein entsprechendes Computerprogrammprodukt, und ein entsprechendes Computerlesbares Speichermedium werden ebenfalls gezeigt.

## Beschreibung

### GEBIET DER ERFINDUNG

Die vorliegende Erfindung betrifft im Allgemeinen die Nutzung von Medizinprodukten. Insbesondere wird die Speicherung von Daten über deren Nutzung in einer, zumindest im derzeit technisch allgemein möglichen und wirtschaftlich sinnvollen Rahmen, fälschungssicheren Art beschrieben.

### HINTERGRUND DER ERFINDUNG

Die folgende Beschreibung des Standes der Technik dient dazu die Erfindung und insbesondere die Vorteile der Erfindung besser verstehen zu können. Es versteht sich, dass jegliche Darlegung des Standes der Technik weder explizites noch implizites Zugeständnis ist, dass dieser Stand der Technik weithin bekannt ist oder Allgemeinwissen auf dem Gebiet der Erfindung darstellt.

Gegenwärtig kann die begrenzte Wiederverwendung von Medizinprodukten, z.B. nur einmalige Benutzung aus Hygiene- oder Kontaminationsgründen, oder nur eine bestimmte Anzahl von Wiederverwendungen aus Abnutzungs- oder Haltbarkeitsgründen, nur schwierig sichergestellt werden. Eine versehentliche Verwendung über die erlaubte Nutzung hinaus kann versehentlich stattfinden, sofern ein korrektes Label, eine Gebrauchsanweisung oder eine Beschriftung und weitere möglicherweise ergriffenen Vorsichtsmaßnahmen für die Produkte nicht eingehalten werden.

Darüber hinaus kann mit dem verfügbaren Stand der Technik, eine unabsichtliche oder vorsätzliche Verwendung über die erlaubte Nutzung hinaus nicht ausgeschlossen und auch im Nachhinein nicht nachverfolgt werden. Das Ermitteln einer solchen unabsichtlichen oder vorsätzlichen Verwendung über die erlaubte Nutzung hinaus kann nur aufgrund von Indizien wie Abnutzungserscheinen und Kundenaussagen stattfinden.

Aktuell vorhandene und genutzte Datenbanklösungen wie SQL, MYSQL, SAP, Oracle etc. erfüllen die Anforderung der Unverfälschbarkeit der Inhalte nur unzureichend. Die Daten in solchen Datenbanken können unter Umständen jederzeit nach Belieben geändert werden, auch wenn gegebenenfalls die Notwendigkeit für Administrationsrechte um solche Änderungen durchzuführen, eine gewisse Sicherheit vermitteln, so ist doch anhand der Daten nicht ersichtlich ob die gespeicherten Daten korrekt sind.

Darüber hinaus sind die Lösungen des Standes der Technik in gewissem Maße anfällig für Angriffe durch Dritte und somit potentiell fälschbar.

Die Aufgabe der Erfindung ist es die oben beschriebenen Nachteile des Standes der Technik zu überwinden, den Stand der Technik zu verbessern, und/oder wenigstens eine nützliche Alternative zum bestehenden Stand der Technik anzubieten.

### ZUSAMMENFASSUNG DER ERFINDUNG

Die vorliegende Erfindung löst die obengenannte Aufgabe durch die Gegenstände der unabhängigen Patentansprüche. Vorteilhafte Weiterbildungen finden sich in den jeweiligen abhängigen Patentansprüchen.

Insbesondere löst die vorliegende Erfindung die Aufgabe durch Erfassen einer Nutzung des Produktes, Erstellen von Nutzungsdaten, die die erfasste Nutzung des Produktes betreffen, und Speichern der Nutzungsdaten in einem Datensatz in einer nachträglich nicht modifizierbaren Form. Unter einer "nachträglich nicht modifizierbaren Form" soll dabei insbesondere auch eine Form einer Speicherung verstanden werden, die unter wirtschaftlichen Gesichtspunkten nicht veränderbar ist, beispielsweise weil ein Aufwand für eine Veränderung unwirtschaftlich groß ist. Zum Speichern der Nutzungsdaten in dem Datensatz wird erfindungsgemäß eine Blockchain-Technologie verwendet, was die nachträgliche Modifikation der Daten gut verhindert. Durch Speicherung des Datensatzes als einen Blockchain-Datensatz kann eine dezentrale Speicherung realisiert werden, wodurch ein gegen den Ausfall einzelner Server gesicherte Speicherung realisiert werden kann. Gerade im Bereich von Medizinprodukten ist eine hohe Zuverlässigkeit von Vorteil. Außerdem muss sichergestellt werden können, dass die Speicherung des Datensatzes und eine Auswertung bereits gespeicherter Datensätze auch dezentral ohne permanente Anbindung an einen Server realisiert werden kann. Dies kann erreicht werden, indem der Datensatz die Blockchain-Technologie verwendet wird, ohne dass gleichzeitig ein Risiko durch nachträgliche Modifizierungen steigt.

Die Nutzungsdaten können auch vom Produkt, das genutzt wird, an einen Rechner oder mehrere Rechner übertragen werden und dort gespeichert werden. Unter Rechner ist hierbei jedwede Art von Datenverarbeitungseinrichtung zu verstehen.

In diesem Fall kann der Blockchain-Datensatz auf dem einem oder den mehreren Rechnern in ein bestehendes Datenkonstrukt, das ebenfalls auf Blockchain basieren kann, integriert werden.

Die Speicherung auf dem Produkt oder den Rechnern kann verschieden ausgeführt werden. Es ist möglich, dass die Speicherorte allesamt nicht-öffentlich, z.B. innerhalb einer Firma oder eines Krankenhauses, angeordnet sind. Die Rechner, können aber auch verteilt, nicht-öffentlich angeordnet sein, z.B. beim Hersteller, Servicepartner und Nutzer des Produktes. Oder aber, die Speicherung wird in einer öffentlich zugänglichen DLT durchgeführt.

Die dabei eingesetzte Datenübertragen kann beispielsweise durch verschiedene Technologien bewerkstelligt werden: Bluetooth, IR, LAN, WLAN, IoT, Mobilfunk, z.B. 2G, 3G, 4G, 5G, GSM und/oder LTE, LoRaWAN (Low Range Wide Area Network). Andere drahtgebundene oder drahtlose Protokolle oder Übertragungstechnologien können jedoch ebenfalls eingesetzt werden. Darüber hinaus ist eine direkte Übertragung oder eine Übertragung über Zwischenstationen, wie z.B. Smart Hubs, Repeater, Router, Switch, Mobile Hotspots, Data Gates, Access Points, und/oder Internet möglich.

In den gespeicherten Datensätzen oder Datenkonstrukten können auch zusätzliche Informationen gespeichert werden. Beispiele hierfür sind Informationen bezüglich Artikel, Seriennummer des Produktes und/oder einer erlaubten Nutzung, wie etwa ein Ablaufdatum, nach dem das Produkt nicht mehr verwendet werden darf, oder eine erlaubte Anzahl von Nutzungen. Diese Informationen können entweder bei der Herstellung des Produktes in das Datenkonstrukt geschrieben werden oder bei der Erfassung der Nutzung miterfasst werden und dann in den Datensatz gespeichert werden. Andere Informationen die gespeichert werden können sind beispielsweise Information bezüglich Daten der Herstellung, Lagerung, Versand, Verkauf des Produktes.

Mit den zusätzlich gespeicherten Daten kann bei einer Anforderung einer Nutzung an das Produkt bestimmt werden, ob diese angeforderte Nutzung erlaubt ist oder eine unerlaubte Nutzung darstellt. Dies geschieht durch Auswertung der in dem Datensatz und/oder dem Datenkonstrukt gespeicherten Daten. Für Medizinprodukte stellt damit die Speicherung in Form einer Blockchain eine besonders vorteilhafte Ausgestaltung dar.

Sollte festgestellt werden, dass die weitere Nutzung eine unerlaubte Nutzung ist, so kann, die unerlaubte Nutzung fälschungssicher festgehalten werden. Alternativ oder zusätzlich kann ein Signal, z.B. akustisch und/oder optisch ausgegeben werden. Weiterhin zusätzlich oder alternativ können Daten übertragen werden, die an einer anderen Vorrichtung ein solches Signal auslösen oder dort eine Benachrichtigung verursacht. Darüber hinaus kann alternativ oder zusätzlich eine Aufforderung ausgegeben werden und das Produkt zur Benutzung erst nach Bestätigung, z.B. durch einen Benutzer, der Aufforderung freigegeben werden. Dies ermöglicht beispielsweise eine Notfallbenutzung, so dass ein eigentlich nicht mehr zu benutzendes Produkt dennoch eingesetzt werden kann. Gründe hierfür können beispielsweise sein, dass kein unbenutztes oder innerhalb der Nutzungsparameter zu benutzendes Produkt verfügbar ist. Schließlich kann alternative hierzu oder zusätzlich das Produkt für die angeforderte weitere Nutzung gesperrt werden, so dass es nicht für die als unerlaubte Nutzung bestimmte Nutzung eingesetzt werden kann. Dies kann die Gesamtsicherheit erhöhen, da ein Produkt möglicher Weise nach Erreichen der erlaubten Nutzungszeiten oder Anzahl von Nutzungen ein Sicherheitsrisiko darstellen kann, da z.B. bei Schneidewerkzeugen die Schneiden potentiell Schaden verursachen.

Weitere Vorteile der Erfindung bestehen in der fälschungssicheren Dokumentation von Nutzung von Produkten. Dadurch kann eine unerlaubte Nutzung festgestellt und durch die Verhinderung einer solchen die allgemeine Sicherheit bei der Benutzung erhöht werden.

### KURZBESCHREIBUNG DER ZEICHNUNG

Die obenstehende Zusammenfassung, sowie weitere Aufgaben, Merkmale und Vorteile der vorliegenden Erfindung werden im Weiteren durch und mit Bezug auf die folgenden Figuren der Zeichnung erläutert, um so ein besseres Verständnis der Erfindung zu ermöglichen. Darin bezeichnen gleiche Bezugszeichen gleiche Elemente.

Es zeigen:
die Figuren 1 bis 4 Flussdiagramme des erfinderischen Verfahrens;
die Figuren 5 bis 8 beispielhafte Ausführungsbeispiele der Erfindung; und
die Figur 9 einen beispielhaften Prozess mit beispielhaften speicherbaren Informationen.

### AUSFÜHRLICHE BESCHREIBUNG

Um bei Produkten, die nur eine bestimmte Anzahl von Nutzungen oder auch nur in einem bestimmten Zeitraum, oder Ähnliches, verwendet werden sollen, diese Begrenzung sicherzustellen, ist ein Nachweis der Nutzungen erforderlich. Idealerweise ist solch ein Nachweis fälschungssicher, um nicht nachträglich Nutzungen hinzuzufügen, zu löschen oder verändern zu können. Um den Aspekt des verifizierten Nachweises zu erfüllen und als Hersteller als auch Kunde eines Produktes (z.B. Qualitätsmanagement eines Krankenhauses, Materialverwaltung von Betrieben, oder Ähnliches) verlässliche Informationen herausgeben zu können, und dadurch die Rechtssicherheit bei kritischen Verwendungen zu erhöhen, wird eine Lösung vorgeschlagen, die eine Unverfälschbarkeit der Daten in Form von Logs sicherstellt und somit die Nutzungen historisch sicher dokumentiert. Für die Nutzung von Produkten interessieren sich in der Regel der Hersteller der Produkte, der/die Anwender der Produkte, also diejenigen die die Nutzung direkt durchführen, aber auch möglicher Weise alle Beteiligten der Lieferkette zwischen Hersteller und Anwender. Dies können Händler sein, aber auch möglicher Weise Zwischenstation wie Materiallagerstellen und zentrale Verwaltungsstellen z.B. bei einem Krankenhausverbund. Auch Hersteller und Anwender sind im Folgenden von dem Begriff Beteiligte erfasst.

Hierbei wird die bevorzugt eine sogenannte Distributed-Ledger-Technology (kurz DLT, englisch für Technik verteilter Kassenbücher) eingesetzt, die im Folgenden kurz erläutert wird. DLT wird für die Dokumentation bestimmter Transaktionen benutzt. Im Gegensatz zum klassischen Ansatz, bei dem ein Hauptbuch in der Regel von nur einer Instanz verwaltet wird, werden hier dezentral beliebig viele prinzipiell gleichgestellte Kopien des Ledgers von unterschiedlichen Parteien unterhalten. Durch geeignete Maßnahmen wird dafür gesorgt, dass neu hinzuzufügende Transaktionen in allen Kopien des Ledgers übernommen werden und dass es zu einer Übereinkunft (Konsensus) über den jeweils aktuellen Stand des Ledgers kommt.

DLT ist eine Form der Blockchain, andere sind z.B. Tangle. Nachfolgend wird der Überbegriff Blockchain genutzt, die Erfindung kann jedoch auch mit jeder anderen Blockchain-Technologie umgesetzt werden.

Diese Lösung hat unter anderem folgende, beispielhafte Vorteile. Ein Nutzer (z.B. Hersteller, Anwender oder Händler, etc.) kann durch Verwendung der Blockchain valide ermitteln und loggen ob ein Produkt öfter als die bestimmte maximale Anzahl von Nutzungen eingesetzt wurde. Beispielsweise kann festgestellt werden, oder ein Single-Use-Artikel (ein Produkt, das z.B. aus hygienischen Gründen nur einmal eingesetzt werden darf) mehrfach eingesetzt wurde. Der Nutzer kann auch sicherstellen das Produkte nur einmalig eingesetzt werden. Dies kann durch eine Sperrung (z.B. bei stromführenden Geräten) oder durch Signalhinweise (z.B. akustische, optische Signale) oder auch eine Aufforderung in einem Dialog eines Rechners geschehen. Solche Hinweise oder Signale können beispielsweise an Rechner, mobile Geräte, Software die auf solchen betrieben werden (z.B. Programme auf Rechner, Apps auf mobilen Geräten), Anzeigen auf Steuergeräten für die Produkte oder andere Informationskanäle gesendet werden. Dazu können verschiedene Transportprotokolle zum Einsatz kommen. Z.B.: SMS, E-Mail, TCP/IP etc, oder Datenübertragung über Bluetooth, IR, LAN, WLAN, IoT, Mobilfunk, z.B. 2G, 3G, 4G, 5G, GSM und/oder LTE, LoRaWAN. Auch kann die Kommunikation entweder direkt oder über Zwischenstationen stattfinden, wie Smart Hubs, Repeater, Router, Switch, Mobile Hotspots, Data Gates, Access Points, und/oder Internet.

Durch die sichere Protokollierung der Nutzungen können alle Beteiligten ihre Qualität sichern bzw. sogar steigern und eine versehentliche Mehrfachverwendung oder zu häufige Verwendung von Produkten vermeiden und gegenüber überwachenden Institutionen oder Behörden valide Daten vorzeigen und Beweise führen.

Eine fälschungssichere Protokollierung ist eine in die Zukunft gewandte Technologie und jeder Beteiligte, der eine solche Protokollierung einsetzt, ist dadurch für die zukünftigen Anforderungen gewappnet. Jeder Beteiligte kann sich als Teilnehmer in einer Blockchain etablieren und somit seine Reputation gegenüber Institutionen und Behörden steigern. Dies ist für die wirtschaftliche Vergabe von Aufträgen unter Umständen ausschlaggebend. Eine Fälschung von Daten kann durch den Einsatz nicht mehr vorkommen, da die Historie über die jeweiligen Blocks abgebildet ist. Darüber hinaus können Anwendungszyklen auch für wiederverwendbare Produkte valide und unabänderlich geloggt werden.

Bisheriger Lösungen umfassen das Loggen bzw. Protokollieren von Nutzungsdaten in herkömmlichen Datenbanken wie etwa mysql, sql, etc. Manche davon sind cloudbasiert. Solche Lösungen weisen in der Regel keine Fälschungssicherheit auf. Möglicher Weise werden die Daten auch von Nutzen selbst in solche Datenbanken eingetragen und unterliegen somit auch Fehlern bei der Datenerfassung und/oder Dateneingabe. Darüber hinaus können Daten in solchen Datenbanken von IT-Administratoren (mit sogenannten Administrationsrechten, also einem erweiterten Zugriff auf die Daten) oder durch ungewollten Zugriffen (z.B. Angriffen) von Dritten geändert werden. Solche Veränderungen können unter Umständen erst relativ spät bemerkt werden, so dass dann auch nur zeitverzögert Gegenmaßnahmen eingeleitet werden können. Dies führt insgesamt zu einem hohen Administrations- und Überwachungsaufwand. Ebenfalls sind betrügerischen Absichten bzgl. Datenbankänderungen und Datenmanipulation mit ausreichend krimineller Energie keine Grenzen gesetzt.

Um die Integrität von Beteiligten gegenüber Institutionen und Behörden zu stärken ist die Nutzung der Blockchain ein vorteilhafter Ansatz.

Produkte mit begrenzter Nutzung, insbesondere Single Use Produkte werden bei den Kunden für den vorgesehenen, z.B. einmaligen, Einsatz bereitgestellt und sind in der Regel entsprechend markiert oder beschrieben. Single Use Produkte können beispielsweise durch ein Symbol einer durchgestrichenen Zahl 2 entsprechend markiert sein.

Dieses Symbol ist beispielsweise zu finden auf Produktverpackungen, Label, auf den Produkten selbst, Gebrauchsanweisungen und weiteren geeigneten ggf. vorgeschriebenen Stellen.

Beispielhaft wird untenstehend die Lösung anhand eines Werkzeuges dargestellt, beschränkt sich aber nicht auf dieses alleine. Vielmehr erstreckt sich die Erläuterung auf jegliche Single Use Produkte oder Produkte mit begrenzter Nutzung.

Generell setzt die Erfindung voraus, dass Produkte elektronisch erfasst und erkannt werden können. Dies ist im Stand der Technik beschrieben, z.B. in DE 102018133503 A1, DE 102018126969 A1, DE 102018121682 A1.

Ebenfalls sind aus dem Stand der Technik sogenannte Smart Hubs, Mobile Hotspots, Data Gates, Access Points, Repeater etc. bekannt, welche eine Verbindung zwischen Geräten und/oder ins Internet bieten.

Dadurch werden eine Grundverfügbarkeit und Erreichbarkeit von Diensten im Internet und/oder bei anderen Beteiligten gewährleistet. Beispiele solcher Vorrichtungen beinhalten Mobile Hotspot über das Mobilnetz (5G, 4G, 3G, Dualband, etc.), Basisgeräte für IoT Devices, Smartphones oder WLAN-Router, die einen Hotspot etablieren können, LoRaWAN, die eine Technik einsetzen, die neben 5G als eine der möglichen Grundlagen für IOT Devices etabliert werden, und ähnliches.

Darüber hinaus wird die Funktionalität beschrieben mit mindestens einer Blockchain kommunizieren zu können und Transaktionen auszuführen bzw. Informationen (in Blöcke) einfügen zu können.

Generell kann der komplette Lebenszyklus eines Produktes, gegebenenfalls mit Unterprozessschritten in der Blockchain protokolliert, d.h. geloggt, werden.

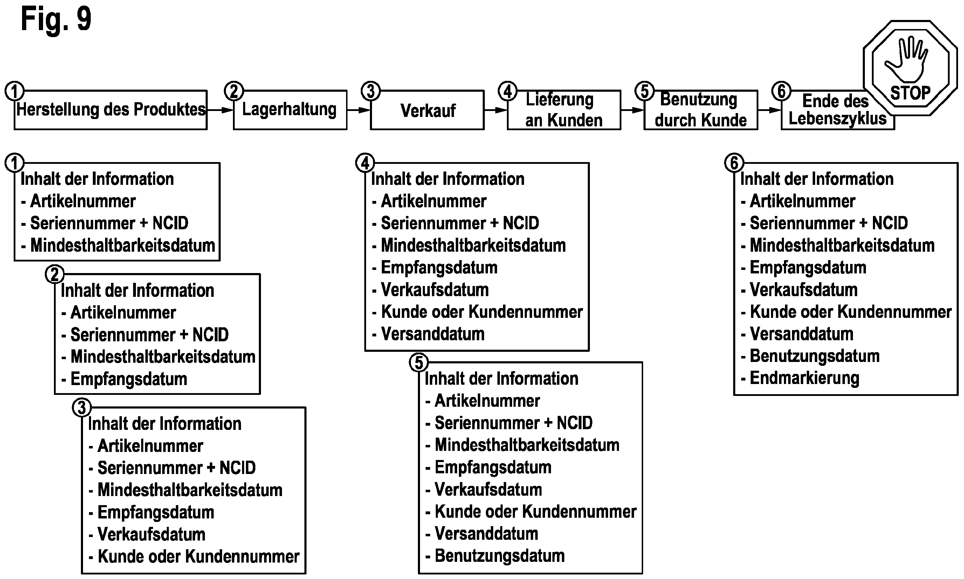

Beispielhaft sind im Folgenden einige mögliche Phasen des Lebenszyklus beschrieben. Diese sind in Figur 9 dargestellt. Zu jeder Phase wird beispielhaft der Inhalt der gespeicherten Daten angezeigt. Die in Figur 9 angegeben Daten dienen lediglich der Veranschaulichung, es können weitere Daten gespeichert werden, wie in dieser Beschreibung ausgeführt, es können auch möglicher Weise Daten entfallen, dem jeweiligen Anwendungsfall entsprechend.

Phase 1 ist die Herstellung des Produktes. Während des Herstellprozesses wird das Produkt gefertigt und final mit Daten bestückt. Das geschieht in Form von Laserbeschriftung oder Beschreiben von Datenspeichern, oder Ähnlichem. Solche Vorgänge sind beispielsweise beschrieben in DE 102018125884. Gemäß der vorliegenden Beschreibung können hierbei beispielsweise eine Artikelnummer, z.B.: GP111SU, eine eindeutige Serialnummer z.B. SN0001020, gegebenenfalls einschließlich einer einmalig vergebenen Datensequenz, z.B. eine NONE COPY IDENTIFICATION, kurz NCID, die optional auch verschlüsselt sein kann, und/oder ein Mindesthaltbarkeitsdatum, z.B.: 2019-09 für September 2019, protokolliert werden. Eine oder mehrere dieser Informationen können auch anzeigen, z.B. dass das Produkt nur zur einmaligen Verwendung, d.h. Single Use, vorgesehen ist. Dies kann beispielsweise über die Buchstabenkombination SU in Artikel oder Seriennummer angezeigt werden. Optional können auch Daten gesondert gespeichert werden, die die Eigenschaft Single Use, Wiederverwendbar, die Anzahl der erlaubten Nutzungen oder den Zeitraum einer erlaubten Benutzung anzeigen. Auf diese Art und Weise ist auch ein Zählen der Anwendungszyklen möglich.

Phase 2 beschreibt die Lagerhaltung des Produktes. Hierbei werden dem bereits protokollierten Datensatz Daten hinzugefügt, die z.B. den Tag des Lagerzugangs und somit die Verkaufsfähigkeit anzeigen. Diese Daten werden zusätzlich zum bereits bestehenden Datenpaket in die Blockchain geschrieben. Weitere Informationen, die den Zugang zum Lager, und die zuvor zurückgelegten Strecken und Zwischenstationen des Produktes beschreiben sind ebenfalls möglich, z.B. Tag der Versendung ab Werk, Angabe der Transportphase, Anzahl und Nummer der Zwischenstationen, Transportdauer, zurückgelegte Entfernung des Produktes, etc.

Phase 3 beschreibt den Verkauf des Produktes. Hierbei werden dem bereits protokollierten Datensatz Daten hinzugefügt, die z.B. die Zeit des Inverkehrsbringen anzeigen. Dies kann als nur Tag oder auch mit zusätzlichen Informationen, wie Uhrzeit, Ort, Zeitzone, etc. geschehen. Diese Daten werden zusätzlich zum bereits bestehenden Datenpaket in die Blockchain geschrieben. Weitere Informationen sind ebenfalls möglich, z.B. Preis, Anzahl und Nummer der Besitzer, Identifikation des Besitzers, Identifikation des Käufers, Identifikation des Verkäufers etc. Die zuvor beschriebenen Identifikationen können auch im Falle von gewünschter Anonymität über verschlüsselte ID-Daten, z.B. Nummern, Symbole, und dergleichen erfolgen, die nur eingeschränkt bekannt sind, z.B. nur dem Hersteller, oder nur öffentlichen Behörden.

Phase 4 beschreibt den Versand des Produktes. Hierbei werden dem bereits protokollierten Datensatz Daten hinzugefügt, die z.B. die Zeit des Versandes anzeigen. Dies kann als nur Tag oder auch mit zusätzlichen Informationen, wie Uhrzeit, Ort, Zeitzone, etc. geschehen. Diese Daten werden zusätzlich zum bereits bestehenden Datenpaket in die Blockchain geschrieben. Weitere Informationen sind ebenfalls möglich, z.B. Tag früherer Versendung, Angabe der Transportphase, Anzahl und Nummer der Zwischenstationen, Transportdauer, zurückgelegte Entfernung des Produktes, Identifikation des Absenders, Identifikation des Empfängers, Transportrestriktionen, wie z.B. zerbrechlich oder keine Luftfracht um einen erhöhten Luftdruck zu vermeiden, etc. Die zuvor beschriebenen Identifikationen können auch im Falle von gewünschter Anonymität über verschlüsselte ID-Daten, z.B. Nummern, Symbole, und dergleichen erfolgen, die nur eingeschränkt bekannt sind, z.B. nur dem Hersteller, oder nur öffentlichen Behörden.

Phase 5 beschreibt die eigentliche Nutzung des Produktes. Hierbei werden dem bereits protokollierten Datensatz Daten hinzugefügt, die z.B. die Zeit der Nutzung anzeigen. Dies kann als nur Tag oder auch mit zusätzlichen Informationen, wie Uhrzeit, Ort, Zeitzone, etc. geschehen. Diese Daten werden zusätzlich zum bereits bestehenden Datenpaket in die Blockchain geschrieben. Weitere Informationen sind ebenfalls möglich, z.B. Ausschreiben zwischengespeicherter Nutzungsdaten im Gerät seit der letzten Verbindung zur Erfassungseinheit, Identifikation der nutzenden Person oder Vorrichtung, die z.B. über ein Identifizieren vor der Nutzung erfasst werden kann, weitere Daten der Verwendung, d.h. Art der Verwendung, Dauer der Verwendung, Anzahl und Nummer der Nutzungen, Anzahl der Ein- und Ausschaltvorgänge oder durchgeführte Aktionen während der Nutzung. Die zuvor beschriebenen Identifikationen können auch im Falle von gewünschter Anonymität über verschlüsselte ID-Daten, z.B. Nummern, Symbole, und dergleichen erfolgen, die nur eingeschränkt bekannt sind, z.B. nur dem Hersteller, dem Eigentümer, oder nur öffentlichen Behörden.

Phase 6 beschreibt das Ende des Lebenszyklus. Es wird, z.B. vom Produkt selbst oder einer anderen Vorrichtung, aufgrund der zuvor gespeicherten Daten, z.B. Anzahl und/oder Nummer der bereits gespeicherten Nutzungen, Tagesdaten der gespeicherten Nutzungen, etc bestimmt, dass das Ende des Lebenszyklus des Produktes erreicht ist.

Das Produkt ist somit als abgelaufen, oder z.B. expired, gekennzeichnet, und somit gilt, dass dieses Produkt entsorgt und nicht nochmals verwendet werden dürfte. Auch der Zeitpunkt der Vornahme der Markierung des Produktes als "expired" kann festgehalten werden.

Sollten entsprechende Daten in der Blockchain für das Produkt gespeichert sein, kann in Phase 5 auch die Nutzung besonders markiert gespeichert werden, z.B. als Off Label Use, und/oder das Produkt für die Nutzung gesperrt werden, falls die technischen Möglichkeiten dazu vorliegen. Würde beispielsweise ein Produkt das als "expired" markiert ist, an einem späteren Tag oder nach einem definierten Zeitraum nochmals in einer Nutzungsumgebung erfasst werden, so kann wie oben erwähnt ein Off Label Use festgestellt werden. Dadurch wird das Patienten- als auch das Anwenderrisiko reduziert.

Die Sperrung des Produktes, bzw, das Zwischenschalten einer Sicherheitsabfrage vor der Nutzung, kann nun für das Produkt vorgeschrieben werden. Bei Geräten mit Stromversorgung kann z.B. die Anschaltung technisch verhindert werden.

Um einen Notbetrieb dennoch zu ermöglichen, könnte ein Nutzer die zwischengeschaltete Aufforderung bestätigen, was wiederum in Phase 5 über zusätzliche Daten in den Datensatz in der Blockchain gespeichert werden könnte. Dies kann wiederum mit der oben beschriebenen Identifikation des Nutzers oder der nutzenden Vorrichtung geschehen. Andernfalls müsste ein Produkt eingesetzt werden, dessen Lebenszyklus noch nicht das Ende erreicht hat.

Für diesen Fall bestehen mehrere alternative Möglichkeiten:

Der Nutzer wird nicht informiert. Die Off Label Nutzung wird lediglich protokolliert. Der entsprechende Beteiligte, z.B. der Betrieb oder die Institution bei dem/der das Produkt in Benutzung ist kann die Produktdaten in der Blockchain einsehen und feststellen, wenn ein Produkt mehrfach verwendet wurde. Gegebenenfalls kann ein Beauftragter für die Qualitätssicherung dann eingreifen und das Produkt aus dem Kreislauf entfernen lassen.

Der Nutzer erhält während der Nutzung, bzw. beim Beginn der Nutzung des konkreten Produktes Informationen angezeigt, die auf das Ende des Lebenszyklus hinweisen. Dies kann über eine Anzeige akustischer oder optischer Art auf dem Produkt oder an einer geeigneten Vorrichtung, die an der Nutzung beteiligt ist, geschehen. Solch geeignete Ausgabeeinheiten können beispielsweise folgende sein: Tablet, Steuergerät, Smart Watch, Display, Bildschirm im Nutzungsraum und alle geeigneten Ausgabegeräte. Gegebenenfalls kann der Hinweis auf erlaubte Nutzung zusammen mit einer Anzeige erfolgen, ob das Produkt für den vorgesehenen Einsatz überhaupt geeignet ist.

Die Ausgabe eines entsprechenden Hinweises kann auch an anderer Stelle erfolgen. Entweder an einer Ausgabevorrichtung an einer zentralen Überwachungsstelle oder auch als Rückmeldung an den Hersteller. Von dort kann dann ein Servicetechniker oder Vertriebsperson an den Kunden entsandt werden, um über die Nutzung zu beraten.

Für den weiteren Verlauf im Lebenszyklus, wie etwa Rücksendung, Wartung, Wiederaufbereitung, Verwertung und/oder Entsorgung, sind gleiche oder ähnliche Daten wie zuvor beschrieben in die Blockchain speicherbar.

Für alle oben beschriebenen beispielhaften Phasen gilt, dass das Produkt für die Speicherung der Daten in der Regel von einem elektronischen Leser erfasst wird. Dies kann z.B. durch ein Einlesen eines Strichcodes, in 2D oder 3D, oder eines NFC (Near Field Communication) Tags auf dem Produkt, der Umverpackung oder der Versandverpackung geschehen. Auch ist eine Zuordnung über die Versandverpackung über eine zwischengelagerte Datenbankabfrage möglich.

Durch diese Methode der Nutzung von DLT/Blockchain Technologie wird Rechtssicherheit gegeben und die Integrität gegenüber Institutionen und Behörden verbessert. Ebenso erhöht sich die Reputation aller Beteiligten, die diese Lösung einsetzen. Diese führt zu verbesserter Stellung bei Händlern, Auftraggebern und Endkunden.

Die DLT bzw. Blockchain kann in verschiedenen Variationen eingesetzt werden. Eine Möglichkeit ist eine sogenannte private Blockchain. Hierbei wird die Blockchain innerhalb eines abgeschlossenen Netzwerks innerhalb eines Betriebes oder Institution betrieben und möglicher Weise auf mehreren Servern redundant gehalten. Diese Server gelten als sogenannte Nodes/Masternodes. Die Blockinhalte sind somit für andere Beteiligte außerhalb des privaten Netzwerkes nicht einsehbar. Dennoch kann eine entsprechende Datenweitergabe erfolgen, um einen entsprechenden Nachweis führen zu können.

Eine weitere Möglichkeit ist eine semi-private Blockchain. Hierbei kann die Blockchain beispielsweise bei zwei oder mehreren Beteiligen geführt und gemeinsam betrieben, d.h. beschrieben werden. Alternativ ist auch ein Nur-Lese-Zugang für einige Beteiligte möglich. Ein Beispiel ist hierbei der Betrieb der geteilten Blockchain bei Nutzer und Hersteller, so dass beispielsweise eine Nachbestellung abgelaufener Produkte automatisiert werden kann. Die Blockinhalte sind somit für weitere Beteiligte außerhalb des semi-privaten Netzwerkes nicht einsehbar. Dennoch kann eine entsprechende Datenweitergabe erfolgen, um einen entsprechenden Nachweis führen zu können.

Eine weitere Möglichkeit ist eine öffentliche Blockchain. Hier wird die Blockchain öffentlich betrieben und alle Beteiligten, evtl. sogar unbeteiligte, d.h. die Öffentlichkeit, können die Transaktionen, d.h. die Blockinhalte einsehen. Die Daten welche darin geschrieben werden, können in Klarschrift oder in verschlüsselter Form abgelegt werden, was wiederum einen gewissen Grad an Anonymität erzeugen kann. Hierzu können z.B.: Masternodes an den verschiedenen Firmenstandorten der Welt betrieben werden. Gegebenenfalls werden Kunden mit ihren eigenen Masternodes in das Netzwerk integriert. Des Weiteren ist eine Adaption an eine bereits vorhandene Blockchain wie Ethereum, NEO, Cardano denkbar um die zu schreibenden Daten dort einzuspeisen.

Darüber hinaus gibt es die Möglichkeit, die Nutzung auch von Produkten zu erfassen und zu speichern, wenn vorübergehend oder dauerhaft keine Verbindung zur verwendeten Blockchain, z.B. über das Internet bei einem Ausfall desselben, besteht. Dies kann beispielsweise mit einer sogenannten Sidechain erfolgen. Dabei kann auf dem Produkt oder einer Vorrichtung, die in kommunikativer Verbindung mit dem Produkt steht, parallel zur eigentlich verwendeten Blockchain, eine sogenannte Sidechain etabliert werden. Dies kann auch beim Feststellen eines Datenkommunikationsfehlers möglicher Weise ad hoc, also ohne Vorbereitung, geschehen. Somit kann lokal eine eigenständige, fortlaufende Blockchain betrieben werden, welche erst einmal lokal Blöcke generiert und entstandene Produktnutzungen lokal protokolliert, d.h. loggt.

Sollte, im Falle einer nur temporär unterbrochenen Verbindung, die Verbindung wiederhergestellt werden, oder im Falle einer dauerhaften fehlenden Verbindung, die Verbindung, z.B. im Rahmen einer Wartung, einer vorbeugenden Instandhaltung, eines Servicetechnikereinsatz, oder einer anderen geplanten Datenübernahme, gezielt hergestellt werden, kann eine Verbindung zur eigentlichen Blockchain, hier dann Main-Blockchain hergestellt werden und die Dateninhalte der Sidechain können auf die Main-Blockchain übertragen werden, bzw in diese integriert werden. Die Datenblöcke der Sidechain werden dann beispielsweise als weitere Blöcke in der Main-Blockchain abgelegt. Diese Variante ermöglicht es auch Produkte, die ohne Datenverbindung, d.h. offline betrieben werden in die Blockchain zu integrieren, wenn auch zeitverzögert. Medizinprodukte können dadurch mit hoher Verfügbarkeit eingesetzt werden, ohne dass auf die genannten Vorteile verzichtet werden muss. Hardwareanforderungen können gering gehalten werden, da lediglich eine Sidechain erzeugt und lokal gespeichert wird. Auf aufwendige Mechanismen zur Replizierung und Synchronisation von klassischen Datenbanken kann verzichtet werden, womit die hohe Verfügbarkeit erreicht werden kann.

Ein weiterer Vorteil der Lösung ist die erhöhte Sicherheit hinsichtlich gefälschter Produkte. Daten wie Seriennummer werden hierbei mit einer eindeutigen Kontrollzahl, die über einen Algorithmus erzeugt wird und beispielsweise aus einem eindeutigen alphanumerischen Zahlencode besteht, für ein Produkt in der Blockchain gespeichert. Wird anschließend ein gefälschtes Produkt mit der gleichen Seriennummer und einer versucht gefälschten Kontrollzahl erkannt, wird dieses Produkt als Fälschung deklariert und der Nutzer, bzw. Kunde oder der aktuell handelnde Beteiligte, informiert und/oder dies in der Blockchain protokolliert. Eine Patientensicherheit kann erhöht werden. Die Erkennung gefälschter Produkte kann auch über andere Verfahren, wie z.B. der Vergleich von Prüfsummen (engl. checksum) oder den Abgleich eines angehängten verschlüsselten Datensatzes (z.B. einer Kontrollzahl) mit einem auf einem Server (z.B. in der Cloud, d.h. auf einem über Datenkommunikation erreichbaren entfernten Speicher) abgelegten Primärschlüssel erfolgen. Hierdurch kann festgestellt werden ob das Produkt authentisch oder eine Fälschung ist. Die verwendete Verschlüsselung selbst kann dann mit dem Primärschlüssel decodiert und mit dem zuvor hinterlegten (z.B. beim Hersteller oder einem anderen Beteiligten in der Lieferkette) Datensatz abgeglichen werden. Somit kann die Echtheit verifiziert werden und der möglicher Weise gefährliche Einsatz von gefälschten Produkten vermieden werden.

Ein Betrieb von Datenbanken schließt die Anwendung von Blockchain Technologie nicht aus. Beide Technologien können zusammen verwendet werden. Blockchain, oder allgemeiner DLT, ist an sich ist relativ ungeeignet, um große Datenmengen zu speichern, da durch die Fortschreibung in Blöcken die Datenmenge erheblich anwächst. Vielmehr ist bei großen Datenmengen eine gemischte Datenbank/Blockchain-Lösung anzustreben. Hierbei werden größere Mengen von Daten in der Datenbank gesichert und abgelegt. Die zugehörige Blockchain hingegen validiert den abgelegten Stand der Daten und garantiert somit die Echtheit und Integrität der Dateninhalte. Beispielsweise wird ein Datensatz in der Datenbank gesichert. Zu dem Datensatz wird eine Prüfsumme (engl.: Checksum) generiert und gesichert. Die Prüfsumme selbst wird ebenfalls in der Blockchain protokolliert um die Datenintegrität unveränderbar festzuhalten. Dadurch können die in der Datenbank gesicherten Daten verifiziert werden.

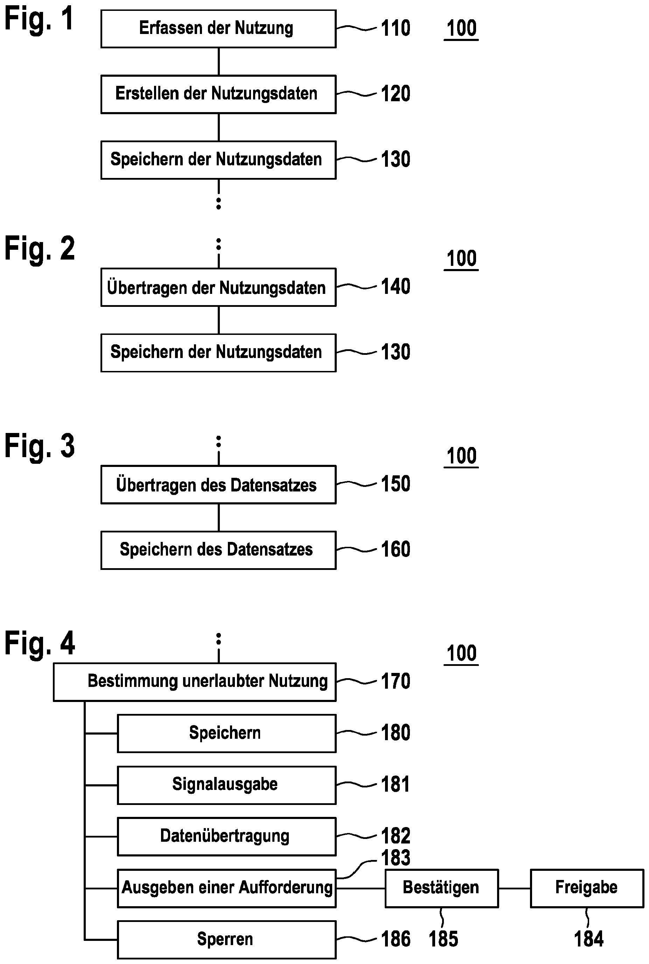

Figur 1 zeigt ein beispielhaftes Verfahren 100 zur Speicherung von Nutzungsdaten eines Produktes. Hierbei wird in einem ersten Schritt 110 zuerst die Nutzung erfasst. Dies kann durch das Produkt oder ein Bauteil davon geschehen, oder durch eine externe Vorrichtung, die die Nutzung erfasst, z.B. dadurch dass das Produkt in Erfassungsreichweite der Vorrichtung ist, z.B. mittels WLAN, Bluetooth, oder einer anderen Funktechnologie, oder dadurch, dass das Produkt der Vorrichtung ein entsprechendes Signal übermittelt. Die Erfassung kann optisch erfolgen, z.B. durch Einlesen eines Strichcodes, in 2D oder 3D auf dem Produkt, oder elektronisch, z.B. durch NFC (Near Field Communication).

Nachfolgend werden in Schritt 120 basierend auf der Erfassung der Nutzungsdaten erstellt, die die erfasste Nutzung des Produktes betreffen. Wie zuvor beschrieben, können hier Daten erfasst werden, wie die Zeit der Nutzung, nur als Tag oder auch mit zusätzlichen Informationen, wie Uhrzeit, Ort, Zeitzone, etc. Die Nutzungsdaten können darüber hinaus eine Identifikation der nutzenden Person oder Vorrichtung, die z.B. über ein Identifizieren vor der Nutzung erfasst werden kann, weitere Daten der Verwendung, d.h. Art der Verwendung, Dauer der Verwendung, Anzahl und Nummer der Nutzungen, Anzahl der Ein- und Ausschaltvorgänge oder durchgeführte Aktionen während der Nutzung enthalten. Die zuvor beschriebenen Identifikationen können auch im Falle von gewünschter Anonymität über verschlüsselte ID-Daten, z.B. Nummern, Symbole, und dergleichen erfolgen, die nur eingeschränkt bekannt sind, z.B. nur dem Hersteller, dem Eigentümer, oder nur öffentlichen Behörden. Schritt 120 kann durch das Produkt oder ein Bauteil davon durchgeführt werden, oder durch eine externe Vorrichtung, die die Nutzung erfasst hat, oder an die die Erfassung mitgeteilt wurde.

In einem nächsten Schritt 130 werden dann die Nutzungsdaten in einem Datensatz in einer nachträglich nicht modifizierbaren Form gespeichert. Die nachträglich nicht modifizierbare Form ist hierbei beispielsweise, wie oben beschrieben, eine DLT wie etwa Blockchain. Gegebenenfalls wird der Datensatz zusätzlich zu bereits bestehenden Datenblöcken in die Blockchain geschrieben. Das Schreiben 130 kann ebenfalls durch das Produkt oder ein Bauteil davon durchgeführt werden, oder durch eine externe Vorrichtung, die die Nutzungsdaten erstellt hat, oder an die die Nutzungsdaten mitgeteilt wurde. Das Schreiben kann auf einem Speicher durchgeführt werden, der im Produkt angeordnet ist, oder in einem Speicher der einer externen Vorrichtung zugeordnet ist.

Figur 2 zeigt, dass das Verfahren 100 nach dem Erstellen 120 und vor dem Speichern 130 zuvor beschriebenes Übertragen 140 der Nutzungsdaten an einen oder mehrere Rechner mit je einem Speicher umfassen kann. In diesem Fall kann das Speichern in Schritt 130 dann von einem oder mehreren Rechnern durchgeführt werden. Auch hier kann das Schreiben auf einem Speicher durchgeführt werden, der im Produkt angeordnet ist, oder in einem Speicher der einer externen Vorrichtung zugeordnet ist.

Figur 3 zeigt, dass das Verfahren 100 in der Form ausgeführt werden kann, dass das Speichern in Schritt 130 von dem Produkt zunächst in einem eigenen Speicher ausgeführt wird und dann einem zuvor beschriebenen Verfahren ein Übertragen 150 des Datensatzes an einen oder mehrere Rechner folgt, die je einen Speicher haben, und sodann der Datensatz von einem oder mehreren Rechner in dessen Speicher gespeichert werden. Da der Datensatz hier bereits aus Schritt 130 die nachträglich nicht modifizierbare Form aufweist, also als DLT oder Blockchain vorliegt, entspricht dies beispielsweise oben ausgeführter Sidechain. Diese könnte dann nach dem Übertragen 150 in ein auf dem empfangenden Rechner in ein bereits bestehendes Datenkonstrukt, d.h. beispielsweise die Main-Blockchain eingefügt werden. Alternativ könnte der Rechner aber auch nur eine einfache Datenbank aufweisen, in der die Sidechain zur späteren Weiterverwendung abgelegt wird.

Figur 4 zeigt, dass dem Verfahren 100 wie zuvor beschrieben, im Ansprechen auf eine Anforderung einer weiteren Nutzung an das Produkt ein Schritt 170 des Bestimmens, dass die weitere Nutzung eine unerlaubte Nutzung darstellt folgen kann. Diese Bestimmung kann beispielsweise durch Auswertung der in dem Datensatz und/oder dem Datenkonstrukt gespeicherten Daten erfolgen, wie zuvor beschrieben. Dieser Schritt kann ebenfalls in dem Produkt oder in einem der zuvor beschriebenen Vorrichtungen, bzw. Rechner erfolgen.

Für den Fall, dass in Schritt 170 eine unerlaubte Nutzung bestimmt wurde, kann im Verfahren einer oder mehrere der folgenden Schritte folgen. In einem Schritt 180 kann die weitere Nutzung, bzw. die Anforderung einer weiteren Nutzung, in dem Datensatz und/oder dem Datenkonstrukt gespeichert werden. In einem Schritt 181 kann ein optisches und/oder akustisches Signal ausgegeben werden. Wie zuvor beschrieben, kann dies beim Produkt oder an einem vom Produkt entfernten Ort geschehen. In einem Schritt 182 können Daten übertragen werden, z.B. an einen Rechner, wie etwa einen der zuvor beschriebenen, wobei die Daten dort dann an einer externen Vorrichtung ein Signal bewirken oder eine Benachrichtigung auslösen. In einem Schritt 183 kann an einen Nutzer eine Aufforderung ausgegeben werden, die dieser zuerst bestätigen muss. Wobei der Erhalt der Freigabe wiederum in Schritt 185 geschieht, und erst danach in Schritt 184 die Freigabe des Produktes bewirkt wird. Wie zuvor beschrieben, kann die Aufforderung und die Freigabe vom Produkt selbst oder von einer zuvor beschriebenen anderen Vorrichtung, wie etwa einem Rechner durchgeführt werden. Schließlich kann in Schritt 186 das Produkt gesperrt werden, so dass eine weitere Nutzung nicht möglich ist.

Die im Zusammenhang mit den Figuren 1 bis 4 beschriebenen Schritte die ein Übertragen von Daten beinhalten, können beispielsweise eine oder mehrere der folgenden Technologien verwenden: Z.B. Bluetooth, IR, LAN, WLAN, IoT, Mobilfunk, z.B. 2G, 3G, 4G, 5G, GSM und/oder LTE, LoRaWAN, und entweder direkt oder über Zwischenstationen, wie Smart Hubs, Repeater, Router, Switch, Mobile Hotspots, Data Gates, Access Points, und/oder Internet.

In den in Zusammenhang mit den Figuren 1 bis 4 beschriebenen Datensätze und Datenkonstrukte, die jeweils als DLT oder Blockchain ausgeführt sein können, können zusätzlich eine oder mehrere Informationen gespeichert werden, aus: Informationen bezüglich Artikel, Seriennummer des Produktes und/oder einer erlaubten Nutzung, wie etwa ein Ablaufdatum, nach dem das Produkt nicht mehr verwendet werden darf, oder eine erlaubte Anzahl von Nutzungen, wobei die Informationen bei der Herstellung des Produktes in das Datenkonstrukt geschrieben werden oder bei der Erfassung in Schritt 110 der Nutzung miterfasst werden und dann in den Datensatz gespeichert werden, oder Information bezüglich Daten der Herstellung, Lagerung, Versand, Verkauf des Produktes.

Nicht gezeigt ist ein System zur Datenverarbeitung, das einen Prozessor umfasst, der das Verfahren wie zuvor beschrieben ausführen kann. Ebenfalls nicht beschrieben ist ein Computerprogrammprodukt und/oder Computerlesbares Speichermedium, das jeweils Befehle umfasst, die bei der Ausführung das Verfahren wie zuvor beschrieben umsetzen.

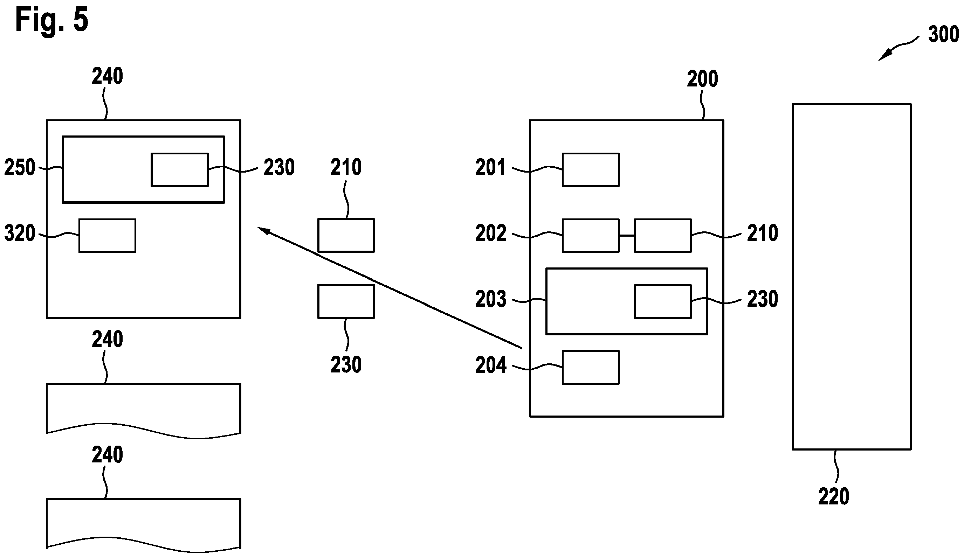

Figur 5 zeigt eine beispielhafte Vorrichtung 200 zur Speicherung von Nutzungsdaten 210 eines Produktes 220. Die Vorrichtung umfasst eine Erfassungseinheit 201, die in oben beschriebener Art die Nutzung des Produktes 220 erfasst.

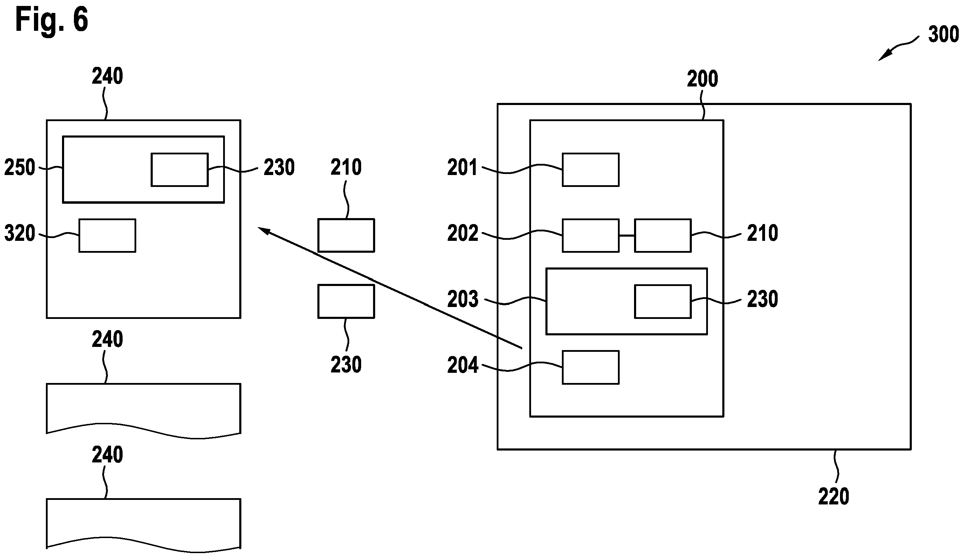

Während in Figur 5 die Vorrichtung 200 eine vom Produkt 220 unabhängige Vorrichtung ist, zeigt Figur 6 ein Ausführungsbeispiel, bei dem die Vorrichtung 200 ein Teil des Produktes 220 ist. Die zuvor und nachstehend beschriebenen weiteren Zusammenhänge sind aber in der Regel gleichermaßen auf die Darstellungen der Figuren 5 und 6 anwendbar.

Stellt die Vorrichtung 200 ein Teil des Produktes dar, so erfasst die Vorrichtung 200 die Nutzung z.B. durch direkte Signalübertragung innerhalb des Produktes 220.

Ist die Vorrichtung 200 kein Teil des Produktes, so kann die Nutzung z.B. dadurch umgesetzt werden, dass erkannt wird, dass das Produkt in Erfassungsreichweite der Vorrichtung ist, z.B. mittels WLAN, Bluetooth, oder einer anderen Funktechnologie, oder dadurch, dass das Produkt der Vorrichtung ein entsprechendes Signal übermittelt. Die Erfassung kann auch optisch erfolgen, z.B. durch Einlesen eines Strichcodes, in 2D oder 3D auf dem Produkt, oder elektronisch, z.B. durch NFC (Near Field Communication).

Des Weiteren umfasst die Vorrichtung 200 eine Datenverarbeitungseinheit 202, die die Nutzungsdaten 210, die die erfasste Nutzung des Produktes 220 betreffen, erstellt. Die Erstellung kann im Produkt oder in einer externen Vorrichtung durchgeführt werden. Wie zuvor beschrieben, können hier Daten erfasst werden, wie die Zeit der Nutzung, nur als Tag oder auch mit zusätzlichen Informationen, wie Uhrzeit, Ort, Zeitzone, etc. Die Nutzungsdaten können darüber hinaus eine Identifikation der nutzenden Person oder Vorrichtung, die z.B. über ein Identifizieren vor der Nutzung erfasst werden kann, weitere Daten der Verwendung, d.h. Art der Verwendung, Dauer der Verwendung, Anzahl und Nummer der Nutzungen, Anzahl der Ein- und Ausschaltvorgänge oder durchgeführte Aktionen während der Nutzung enthalten. Die zuvor beschriebenen Identifikationen können auch im Falle von gewünschter Anonymität über verschlüsselte ID-Daten, z.B. Nummern, Symbole, und dergleichen erfolgen, die nur eingeschränkt bekannt sind, z.B. nur dem Hersteller, dem Eigentümer, oder nur öffentlichen Behörden.

Die Vorrichtung 200 umfasst weiterhin eine Speichereinheit 203, die die Nutzungsdaten 210 in einem Datensatz 230 in einer nachträglich nicht modifizierbaren Form speichert. Die nachträglich nicht modifizierbare Form ist hierbei beispielsweise, wie oben beschrieben, eine Blockchain. Gegebenenfalls wird der Datensatz zusätzlich zu bereits bestehenden Datenblöcken in die Blockchain geschrieben. Die Speichereinheit kann Teil des Produktes oder Teil einer externen Vorrichtung, die die Nutzungsdaten erstellt hat, oder an die die Nutzungsdaten mitgeteilt wurde. Das Speichern kann auf einem Speicher durchgeführt werden, der im Produkt angeordnet ist, oder in einem Speicher der einer externen Vorrichtung zugeordnet ist.

Weiter ist zu sehen, dass die Vorrichtung 200 Teil eines Systems 300 sein kann, wobei das System 300 weiter einen oder mehrere Rechner 240 umfasst, mit je einem Speicher 250. Die Vorrichtung 200 weist dann eine Übertragungseinheit 204 auf, die die Nutzungsdaten 210 an einen oder mehrere der Rechner 240 überträgt. Die empfangenden Rechner speichern dann in den jeweiligen Speichern 250, wie zuvor beschrieben, die Nutzungsdaten 210 in einem Datensatz 230 in einer nachträglich nicht modifizierbaren Form, und zwar einer DLT oder Blockchain.

Bei einem derartigen System 300 kann die Vorrichtung 200 wiederum als Teil des Produktes 220 ausgeführt sein, oder extern, wie in Figur 5 bzw. Figur 6 zu sehen ist. Die Übertragungseinheit 204 kann auch den in der Vorrichtung 200 erstellten Datensatz 230 an den einen oder mehreren Rechner 240 übertragen.

In anderen Worten, wenn die Vorrichtung 200 aus den Nutzungsdaten 210 den Datensatz 230 erstellt, dann kann dieser Datensatz 230 von der Übertragungseinheit 204 an den oder die Rechner 240 übertragen werden um dort oder an anderer Stelle gespeichert zu werden. Wenn aber der oder die Rechner 240 aus den Nutzungsdaten 210 den Datensatz 230 erzeugen, dann werden die Nutzungsdaten 210 von der Übertragungseinheit 204 an den oder die Rechner 240 übertragen werden um dort oder an anderer Stelle in einem Datensatz 230 gespeichert zu werden.

Wird an den oder die Rechner 240 der fertige Datensatz 230 übertragen, so kann der oder die Rechner 240 eine Berechnungseinheit 241 aufweisen, die den übertragenen Datensatz 230 in ein bestehendes Datenkonstrukt 280, das ebenfalls nicht nachträglich modifizierbar ist, d.h. das ebenfalls beispielsweise in DLT oder als Blockchain vorliegt, einzufügen.

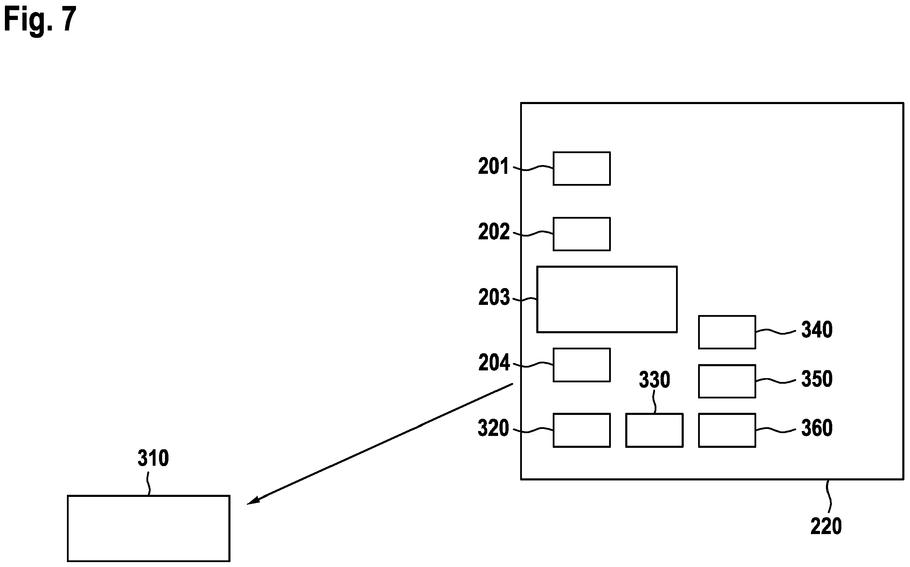

Einer der Rechner 240 oder, wie in Figur 7 gezeigt, die Vorrichtung 200 kann eine Bestimmungseinheit 320 aufweisen, die im Ansprechen auf eine Anforderung einer weiteren Nutzung bestimmen kann, dass die weitere Nutzung eine unerlaubte Nutzung darstellt. Diese Bestimmung kann beispielsweise durch Auswertung der in dem Datensatz 230 und/oder dem Datenkonstrukt 280 gespeicherten Daten erfolgen, wie zuvor beschrieben.

Für den Fall, dass die Bestimmungseinheit 320 eine unerlaubte Nutzung bestimmt, kann einer oder mehrere der Rechner 240, wie in Figuren 5, 6 und 8 gezeigt, oder die Vorrichtung 200, wie in Figur 7 gezeigt, eines oder mehrere der folgenden Elemente aufweisen, bzw. in folgender Weise angepasst sein. Die Vorrichtung 200 oder das System 300 können die weitere Nutzung in dem Datensatz 230 und/oder dem Datenkonstrukt 280 speichern, falls eine unerlaubte Nutzung durch die Bestimmungseinheit 320 bestimmt wurde. Eine Signaleinrichtung 330 kann ein optisches und/oder akustisches Signal ausgeben, falls eine unerlaubte Nutzung durch die Bestimmungseinheit 320 bestimmt wurde. Wie zuvor beschrieben, kann dies beim Produkt oder an einem vom Produkt entfernten Ort geschehen. Die Übertragungseinheit 204 kann Daten, die an einer externen Vorrichtung 310 ein Signal bewirken oder eine Benachrichtigung auslösen, übertragen, falls eine unerlaubte Nutzung durch die Bestimmungseinheit 320 bestimmt wurde. Eine Ausgabeeinrichtung 340, kann eine Aufforderung ausgeben, und eine Freigabeeinrichtung 350, kann im Ansprechen auf ein Erhalten einer Nutzer-Bestätigung als Antwort auf die Aufforderung, das Produkt für die angeforderte weitere Nutzung freigeben, falls eine unerlaubte Nutzung durch die Bestimmungseinheit 320 bestimmt wurde. Eine Sperreinrichtung 360 kann das Produkt 220 sperren, so dass eine weitere Nutzung nicht möglich ist, falls eine unerlaubte Nutzung durch die Bestimmungseinheit 320 bestimmt wurde.

Die im Zusammenhang mit den Figuren 5 bis 8 beschriebenen Element die das Übertragen von Daten beinhalten, können eine oder mehrere der folgenden Technologien beispielsweise verwenden: Bluetooth, IR, LAN, WLAN, IoT, Mobilfunk, z.B. 2G, 3G, 4G, 5G, GSM und/oder LTE, LoRaWAN, und entweder direkt oder über Zwischenstationen, wie Smart Hubs, Repeater, Router, Switch, Mobile Hotspots, Data Gates, Access Points, und/oder Internet.

In den in Zusammenhang mit den Figuren 5 bis 8 beschriebenen Datensätze 230 und Datenkonstrukte 280, die jeweils als DLT oder Blockchain ausgeführt sein können, können zusätzlich eine oder mehrere Informationen gespeichert werden, aus: Informationen bezüglich Artikel, Seriennummer des Produktes und/oder einer erlaubten Nutzung, wie etwa ein Ablaufdatum, nach dem das Produkt nicht mehr verwendet werden darf, oder eine erlaubte Anzahl von Nutzungen, wobei die Informationen bei der Herstellung des Produktes in das Datenkonstrukt geschrieben werden oder bei der Erfassung der Nutzung miterfasst werden und dann in den Datensatz gespeichert werden, oder Information bezüglich Daten der Herstellung, Lagerung, Versand, Verkauf des Produktes.

Eine beispielhafte Ausgestaltung der Vorrichtung aus Figur 5 umfasst
- ein chirurgisches Motorensystem mit einem Handstück und einem Steuergerät
- und ein Werkzeug, welches mit dem Handstück koppelbar ist,

Das Werkzeug bildet das das Produkt 220 aus. Das Werkzeug kann beispielsweise als ein chirurgischer Fräser ausbildet sein, der für die Bearbeitung von Knochen vorgesehen ist. Das Werkzeug ist mit einem QR-Code als ID-Element versehen, welches zumindest eine Artikelnummer und eine Seriennummer umfasst. Alternativ kann das Werkzeug als ID-Element auch einen RFID-Tag, einen NFC-Tag oder Strichcodes aufweisen. Das ID-Element enthält zumindest eine Seriennummer, die bei einer Ausgestaltung als RFID- oder NFC-Tag elektronisch und bei einer Ausgestaltung als QR-Code oder Strichcode optisch auslesbar ist.

Das Handstück umfasst die Erfassungseinheit 201. Die Erfassungseinheit ist in diesem Ausführungsbeispiel als eine optische Leseeinheit ausgebildet, die dazu vorgesehen ist, den QR-Code auf dem Werkzeug auszulesen. Alternativ kann die Erfassungseinheit auch als ein RFID-Leser oder ein NFC-Leser ausgebildet sein. Die Erfassungseinheit 201 ist in dem Handstück angeordnet. Wird das Werkzeug in das Handstück eingesteckt oder mit dem Handstück verbunden, erfasst die Erfassungseinheit 201 das ID-Element und bestimmt zumindest die Seriennummer.

Das Steuergerät umfasst die Datenverarbeitungseinheit 202, die Speichereinheit 203 und die Übertragungseinheit 204. Das Handstück ist in einem betriebsbereitem Zustand mit dem Steuergerät verbunden. Wird das Werkzeug mit dem Handstück gekoppelt, liest die Erfassungseinheit 201 das ID-Element aus. Die Datenverarbeitungseinheit 202 erstellt mittels der aus dem ID-Element bestimmten Seriennummer und sowie ggf. weiterer, während eines Betriebs erfassten Daten, die Nutzungsdaten 210. Die Nutzungsdaten werden dann als nachträglich nicht modifizierbarer Datensatz in der Speichereinheit 203 gespeichert.

Die Übertragungseinheit 204 ist dazu vorgesehen, das Steuergerät mit dem Rechner 240 zu verbinden. Der Rechner 240 ist vorzugsweise als ein lokaler Server ausgebildet, beispielsweise als Intranet-Server für ein Krankenhaus. Um das Steuergerät mit dem lokalen Server zu verbinden, umfasst das System 300 nicht näher dargestellte Access-Points. Die Übertragungseinheit 204 ist für eine drahtlose Verbindung mit den Access-Points vorgesehen. Der Rechner 240 und die Access-Points können drahtgebunden, beispielsweise mittels einer LAN-Verbindung, oder drahtlos mit dem Rechner 240 verbunden sein.

Der Server umfasst die Bestimmungseinheit 320. Die Bestimmung, ob eine Nutzung des Produkts 200 erlaubt oder nicht-erlaubt ist, erfolgt zentral auf dem Server 200. Erkennt die Bestimmungseinheit 320 eine nicht-erlaubte Nutzung, sendet der Rechner 240 eine entsprechende Information an die Vorrichtung 200. In der Ausgestaltung als Steuergerät weist die Vorrichtung 200 ein Display auf, auf dem die Information dargestellt werden kann. In der Vorrichtung können verschiedene Funktionen zur Verarbeitung der Information über eine nicht-erlaubte Nutzung hinterlegt sein. Erkennt die Bestimmungseinheit 320 beispielsweise, dass das Werkzeug ein Single-Use-Werkzeug ist, das bereits verwendet wurde, stellt das Steuergerät die entsprechende Information dar. Ebenfalls denkbar ist, dass das Steuergerät die Benutzung des Werkzeugs sperrt, wobei Funktionen vorgesehen sein können, die einen Notbetrieb zulassen.

Eine beispielhafte Ausgestaltung der Vorrichtung 200 aus Figur 6 ist eine medizinischen Pumpe, beispielsweise eine Infusionspump. Die Nutzungsdaten 210, die in dem Datensatz 230 in einer nachträglich nicht modizifierbaren Form gespeichert werden, können beispielsweise Informationen zu durchgeführten Service-Checks sein. Zusätzlich wird bei jeder Nutzung der Vorrichtung 200 ein Datensatz 230 erstellt, der Nutzungsdaten 210 über die Inbetriebnahme enthält. Erkennt die Bestimmungseinheit 320, dass der Datensatz 230 mit Nutzungsdaten 210 über die Inbetriebnahme zu einer Vorrichtung gehört, bei welche der letzte Datensatz 230 mit Nutzungsdaten 210 zu einem durchgeführten Service-Check abgelaufen ist, kann die als medizinische Pumpe ausgebildete Vorrichtung 220 ein Signal ausgeben, dass der Service-Check abgelaufen ist.

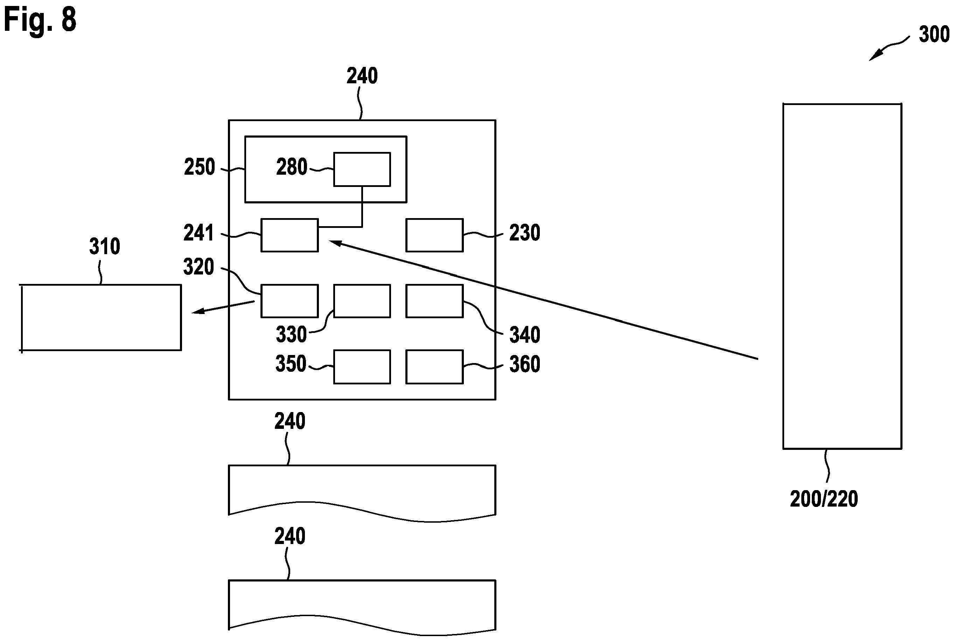

Eine beispielhafte Ausgestaltung für Fig. 8 ist beispielsweise die Ausgestaltung des Produkts 220 als ein Infusionsbeutel. Die Vorrichtung 200 kann in einer solchen Ausgestaltung als eine Halterung für den Infusionsbeutel ausgebildet sein. Alternativ ist es aber auch denkbar, die Vorrichtung 200 als eine Handscanner oder ähnliches auszubilden, wie er beispielsweise bei einem Warenein- oder Ausgang in einem Lager genutzt wird. Die Vorrichtung 200 ist dazu vorgesehen, zumindest eine Identifikationsnummer des Produkts 220 zu erfassen, wie beispielsweise eine Chargennummer. Je nach Ausgestaltung der Vorrichtung 200 können dabei unterschiedliche Verfahren eingesetzt werden. In der Ausgestaltung als Handscanner kann die Vorrichtung für eine optische Erkennung von einem Barcode oder einen QR-Code vorgesehen sein. In der Ausgestaltung als Halterung kann die Vorrichtung für das Auslesen eines NFC- oder RFID-Tags vorgesehen sein.

Bei solchen Produkten wird insbesondere eine Herstellung, Lagerung und ein Transport überwacht. Wird nachträglich festgestellt, dass dabei ein Umstand aufgetreten ist, der eine Sperrung des Produkts 220 erfordert, wird in dem Datenkonstrukt 280 ein Datensatz 230 gespeichert, der diese Information für das Produkt enthält. Die Vorrichtung 220 ist mit dem Rechner 240 verbunden. Ist die Vorrichtung 220 ein Handscanner, kann der Rechner 240 beispielsweise ein Computer eines Lagerverwaltungssystems sein. Ist die Vorrichtung 220 eine Halterung, kann der Rechner 240 beispielsweise Teil eines Patientenüberwachungssystems sein. Insbesondere in einer solchen Ausgestaltung ist die von dem Rechner 240 getrennt ausgeführte Vorrichtung 310 vorteilhaft, um sicherzustellen, dass der Benutzer über die Sperrung des Produkts 220 zu informieren. Die Vorrichtung 310 kann beispielsweise als eine LED ausgebildet sein, durch die die Sperrung signalisiert wird.

## Patentansprüche

1. Computer-implementiertes Verfahren (100) zur Speicherung von Nutzungsdaten (210) eines Produktes (220), wobei das Verfahren (100) umfasst:
Erfassen (110) einer Nutzung des Produktes (220);
Erstellen (120) der Nutzungsdaten (210), die die erfasste Nutzung des Produktes (220) betreffen; und
Speichern (130) der Nutzungsdaten (210) in einem Datensatz (230) in einer nachträglich nicht modifizierbaren Form, wobei der Datensatz (230) eine Blockchain-Technologie verwendet.

2. Verfahren (100) gemäß Anspruch 1, wobei das Verfahren (100) nach dem Erstellen (120) und vor dem Speichern (130) weiterhin umfasst:
Übertragen (140) der Nutzungsdaten (210) an mindestens einen Rechner (240), die jeweils einen Speicher (250) umfassen; und
wobei das Speichern (130) von dem mindestens einem Rechner (240) in dem jeweiligen Speicher (250) ausgeführt wird.

3. Verfahren (100) gemäß Anspruch 1 oder 2, wobei das Speichern (130) von dem Produkt (220) in einem eigenen Speicher (245) ausgeführt wird wobei das Verfahren (100) weiterhin umfasst:
Übertragen (150) des Datensatzes (230) an mindestens einen Rechner (240), die jeweils einen Speicher (250) umfassen; und
Speichern (160) des Datensatzes (230) von dem mindestens einem Rechner (240) in dem jeweiligen Speicher (250).

4. Verfahren (100) gemäß Anspruch 3, wobei der Datensatz (230) des Produktes (220) nach dem Übertragen (150) des Datensatzes (230) in dem mindestens einen Rechner (240) in ein bestehendes Datenkonstrukt (280), das ebenfalls nicht nachträglich modifizierbar ist, eingefügt wird.

5. Verfahren (100) gemäß Anspruch 4, wobei das Datenkonstrukt (280) eine Blockchain-Technologie verwendet.

6. Verfahren (100) gemäß einem der Ansprüche 1 bis 5, wobei die Blockchain-Technologie in einer der folgenden Formen ausgeführt ist:
Blockchain, die nur in Speichern (250) von nicht-öffentlichen Rechnern (240) eines Verbundes (260), abgelegt ist;
Blockchain, die in Speichern (250) von nicht-öffentlichen Rechnern (240) von mindestens zwei Verbünden (260, 270), abgelegt ist; oder
Blockchain, die in Speichern (250) von öffentlichen Rechnern (240), abgelegt ist.

7. Verfahren (100) gemäß einem der Ansprüche 2 bis 6, wobei einer oder mehrere der Schritte Übertragen (140) der Nutzungsdaten und Übertragen (150) des Datensatzes (230) eine oder mehrere der folgenden Technologien beispielsweise verwendet: Bluetooth, IR, LAN, WLAN, IoT, Mobilfunk, z.B. 2G, 3G, 4G, 5G, GSM und/oder LTE, LoRaWAN, und entweder direkt oder über Zwischenstationen, wie Smart Hubs, Repeater, Router, Switch, Mobile Hotspots, Data Gates, Access Points, und/oder Internet übertragen werden.

8. Verfahren (100) gemäß einem der Ansprüche 1 bis 7, wobei in dem Datensatz (230) und/oder dem Datenkonstrukt (280) zusätzlich eine oder mehrere Informationen gespeichert werden, aus:
Informationen bezüglich Artikel, Seriennummer des Produktes und/oder einer erlaubten Nutzung, wie etwa ein Ablaufdatum, nach dem das Produkt nicht mehr verwendet werden darf, oder eine erlaubte Anzahl von Nutzungen, wobei die Informationen bei der Herstellung des Produktes (220) in das Datenkonstrukt (280) geschrieben werden oder bei der Erfassung (110) der Nutzung miterfasst werden und dann in den Datensatz (230) gespeichert werden;
Information bezüglich Daten der Herstellung, Lagerung, Versand, Verkauf des Produktes (220).

9. Verfahren (100) gemäß einem der Ansprüche 1 bis 8, wobei das Produkt (220) ein Medizinprodukt ist.

10. Verfahren (100) gemäß einem der Ansprüche 1 bis 9, weiterhin umfassend:
im Ansprechen auf eine Anforderung einer weiteren Nutzung an das Produkt (220): Bestimmen (170), in dem Produkt (220) oder einem Rechner (240), dass die weitere Nutzung eine unerlaubte Nutzung darstellt, durch Auswertung der in dem Datensatz (230) und/oder dem Datenkonstrukt (280) gespeicherten Daten.

11. Verfahren (100) gemäß Anspruch 10, weiterhin umfassend:
falls in Schritt (170) eine unerlaubte Nutzung bestimmt wurde, Ausführen eines oder mehrere der folgenden Schritte:
Speichern (180) der weiteren Nutzung in dem Datensatz (230) und/oder dem Datenkonstrukt (280);
Ausgeben (181) eines optischen und/oder akustischen Signals;
Übertragen (182) von Daten, die an einer externen Vorrichtung (310) ein Signal bewirken oder eine Benachrichtigung auslösen;
Ausgeben (183) einer Aufforderung, und Freigabe (184) der angeforderten weiteren Nutzung erst nach Erhalten (185) einer Nutzer-Bestätigung;
Sperren (186) des Produktes (220), so dass eine weitere Nutzung nicht möglich ist.

12. System zur Datenverarbeitung, umfassend einen Prozessor, der so angepasst ist, dass er das Verfahren nach einem der Ansprüche 1 bis 11ausführt.

13. Computerprogrammprodukt, umfassend Befehle, die bei der Ausführung des Programms durch einen Computer diesen veranlassen, das Verfahren nach einem der Ansprüche 1 bis 11 auszuführen.

14. Computerlesbares Speichermedium, umfassend Befehle, die bei der Ausführung durch einen Computer diesen veranlassen, das Verfahren nach einem der Ansprüche 1 bis 11 auszuführen.

15. Vorrichtung (200) zur Speicherung von Nutzungsdaten (210) eines Produktes (220), **gekennzeichnet durch**:
eine Erfassungseinheit (201), die dazu eingerichtet ist eine Nutzung des Produktes (220) zu erfassen;
eine Datenverarbeitungseinheit (202), die dazu eingerichtete ist, die Nutzungsdaten (210), die die erfasste Nutzung des Produktes (220) betreffen, zu erstellen; und
eine Speichereinheit (203), die dazu eingerichtet ist, die Nutzungsdaten (210) in einem Datensatz (230) in einer nachträglich nicht modifizierbaren Form zu speichern,
wobei der Datensatz (230) eine Blockchain-Technologie verwendet.

16. System (300) umfassend
eine Vorrichtung (200) gemäß Anspruch 15, und
mindestens einen Rechner (240), die jeweils einen Speicher (250) umfassen, wobei die Vorrichtung (200) weiterhin umfasst:
eine Übertragungseinheit (204), die dazu eingerichtet ist, die Nutzungsdaten (210) an den mindestens einen Rechner (240), zu übertragen; und
wobei die jeweiligen Speicher (250) dazu eingerichtet sind, die Nutzungsdaten (210) in einem Datensatz (230) in einer nachträglich nicht modifizierbaren Form zu speichern.

17. System (300) umfassend
eine Vorrichtung (200) gemäß Anspruch 15, und
mindestens einen Rechner (240), die jeweils einen Speicher (250) umfassen,
wobei die Vorrichtung (200) als Teil des Produktes (220) ausgeführt ist, und weiterhin umfasst:
eine Übertragungseinheit (204), die dazu eingerichtet ist, den Datensatzes (230) an den mindestens einen Rechner (240) zu übertragen; und
wobei die jeweiligen Speicher (250) dazu eingerichtet sind, den Datensatz (230) zu speichern,
wobei das Datenkonstrukt (280) eine Blockchain-Technologie verwendet.

18. System (300) gemäß Anspruch 17, wobei der mindestens eine Rechner (240) weiterhin eine Berechnungseinheit (241) aufweist, die dazu eingerichtet ist, den von der Vorrichtung (200) übertragenen Datensatz (230) in ein bestehendes Datenkonstrukt (280), das ebenfalls nicht nachträglich modifizierbar ist, einzufügen.

19. Vorrichtung (200) oder System (300) gemäß einem der Ansprüche 15 bis 18, wobei die DLT in einer der folgenden Formen ausgeführt ist:
Blockchain, die nur in Speichern (250) von nicht-öffentlichen Rechnern (240) eines Verbundes, abgelegt ist;
Blockchain, die in Speichern (250) von nicht-öffentlichen Rechnern (240) von mindestens zwei Verbünden, abgelegt ist; oder
Blockchain, die in Speichern (250) von öffentlichen Rechnern (240), abgelegt ist.

20. System (300) gemäß einem der Ansprüche 17 bis 19, wobei die Übertragungseinheit (204) eine oder mehrere der folgenden Technologien verwendet: Bluetooth, IR, LAN, WLAN, IoT, Mobilfunk, z.B. 2G, 3G, 4G, 5G, GSM und/oder LTE, LoRaWAN, und entweder direkt oder über Zwischenstationen, wie Smart Hubs, Repeater, Router, Switch, Mobile Hotspots, Data Gates, Access Points, und/oder Internet Daten überträgt.

21. Vorrichtung (200) oder System (300) gemäß einem der Ansprüche 15 bis 20, die weiterhin dazu eingerichtet sind in dem Datensatz (230) und/oder dem Datenkonstrukt (280) zusätzlich eine oder mehrere Informationen zu speichern, aus:
Informationen bezüglich Artikel, Seriennummer des Produktes und/oder einer erlaubten Nutzung, wie etwa ein Ablaufdatum, nach dem das Produkt nicht mehr verwendet werden darf, oder eine erlaubte Anzahl von Nutzungen, wobei die Informationen bei der Herstellung des Produktes (220) in das Datenkonstrukt (280) geschrieben werden oder bei der Erfassung der Nutzung miterfasst werden und dann in den Datensatz (230) gespeichert werden;
Information bezüglich Daten der Herstellung, Lagerung, Versand, Verkauf des Produktes (220).

22. Vorrichtung (200) oder System (300) gemäß einem der Ansprüche 15 bis 21, weiterhin umfassend:
eine Bestimmungseinheit (320), die dazu eingerichtet ist, im Ansprechen auf eine Anforderung einer weiteren Nutzung an das Produkt (220), durch Auswertung der in dem Datensatz (230) und/oder dem Datenkonstrukt (280) gespeicherten Daten zu bestimmen, dass die weitere Nutzung eine unerlaubte Nutzung darstellt.

23. Vorrichtung (200) oder System (300) gemäß Anspruch 22,
wobei die Vorrichtung (200) oder System (300) weiterhin dazu eingerichtet ist, die weitere Nutzung in dem Datensatz (230) und/oder dem Datenkonstrukt (280) zu speichern, falls eine unerlaubte Nutzung durch die Bestimmungseinheit (320) bestimmt wurde;
wobei die Vorrichtung (200) oder System (300) weiterhin eine Signaleinrichtung (330) aufweist, die dazu eingerichtet ist, ein optisches und/oder akustisches Signal auszugeben, falls eine unerlaubte Nutzung durch die Bestimmungseinheit (320) bestimmt wurde;
wobei die Übertragungseinheit (204) weiterhin dazu eingerichtet ist, Daten, die an einer externen Vorrichtung (310) ein Signal bewirken oder eine Benachrichtigung auslösen, zu übertragen, falls eine unerlaubte Nutzung durch die Bestimmungseinheit (320) bestimmt wurde;
wobei die Vorrichtung (200) oder System (300) weiterhin aufweist eine Ausgabeeinrichtung (340), die dazu eingerichtet ist, eine Aufforderung auszugeben, und eine Freigabeeinrichtung (350), die dazu eingerichtet ist, im Ansprechen auf ein Erhalten einer Nutzer-Bestätigung als Antwort auf die Aufforderung, das Produkt für die angeforderte weitere Nutzung freizugeben, falls eine unerlaubte Nutzung durch die Bestimmungseinheit (320) bestimmt wurde; und/oder
wobei die Vorrichtung (200) oder System (300) weiterhin eine Sperreinrichtung (360) aufweist, die dazu eingerichtet ist, das Produkt (220) zu sperren, so dass eine weitere Nutzung nicht möglich ist, falls eine unerlaubte Nutzung durch die Bestimmungseinheit (320) bestimmt wurde.
